# EUROPÄISCHE PATENTSCHRIFT

(11) **EP 0 356 863 B1**
(45) Veröffentlichungstag und Bekanntmachung des Hinweises auf die Patenterteilung: **06.04.1994**
(21) Anmeldenummer: 89115367.8
(22) Anmeldetag: 19.08.1989
(51) Int. Cl.: C07C 35/08, C07C 29/86

(54) **Verfahren zur Abtrennung von Cyclohexanol**
Process for the separation of cyclohexanol
Procédé de séparation du cyclohexanol

(30) Priorität: 27.08.1988 DE 3829143
(43) Veröffentlichungstag der Anmeldung: 07.03.1990
(73) Patentinhaber: BASF Aktiengesellschaft, 67063 Ludwigshafen (DE)
(72) Erfinder: Gosch, Hans-Juergen, Dr., D-6702 Bad Duerkheim (DE); Fischer, Rolf, Dr., D-6900 Heidelberg (DE)

(56) Entgegenhaltungen:
- EP-A- 0 227 931
- JP-A-62 103 033

## Beschreibung

Aus der europäischen Patentanmeldung 123 713 ist ein Verfahren bekannt, bei dem man Cyclohexanol durch Hydratisieren von Cyclohexen bei einer Temperatur von 50 bis 200°C in 5 bis 80 Gew.%igen Lösungen von aromatischen Sulfonsäuren in Wasser erhält. Um Korrosion zu verhindern, wird die Umsetzung z.B. in Gegenwart von Heteropolysäuren, Salzen oder Oxiden des Molybdäns, Wolframs oder Vanadins durchgeführt. Das durch Hydratisierung von Cyclohexen gebildete Cyclohexanol löst sich überwiegend in der aromatische Sulfonsäuren enthaltenden wäßrigen Phase und wird auch durch überschüssiges Cyclohexen nur zum geringen Teil aus dieser abgetrennt.

Entsprechend der japanischen Offenlegungsschrift 103 033/1987 werden zur Extraktion von Cyclohexanol aus aromatischen Sulfonsäure enthaltenden wäßrigen Lösungen aliphatische, cycloaliphatische und aromatische Kohlenwasserstoffe empfohlen. Hierbei sind jedoch erhebliche Mengen Extraktionsmittel erforderlich, um eine wirkungsvolle Extraktion zu erreichen. Dies ist technisch sehr aufwendig.

Nach einem in der europäischen Patentanmeldung 227 931 beschriebenen Verfahren wird die Hydratisierung von Cyclohexen in wäßrigen Lösungen von aromatischen Sulfonsäuren, die mindestens eine Hydroxylgruppe am aromatischen Ring haben, durchgeführt und anschließend mit aromatischen Kohlenwasserstoffen extrahiert. Hierbei gelingt es zwar die Menge an Extraktionsmitteln zu senken; nachteilig ist jedoch, daß durch die Verwendung der Hydroxylgruppen enthaltenden aromatischen Sulfonsäuren die Ausbeute und Selektivität an Cyclohexanol erheblich vermindert wird.

Es war deshalb die technische Aufgabe gestellt, die Abtrennung von Cyclohexanol aus wäßrigen aromatischen Sulfonsäurelösungen, wie sie bei der Hydratisierung von Cyclohexen in Gegenwart von aromatischen Sulfonsäuren anfallen, zu verbessern.

Diese Aufgabe wird gelöst in einem Verfahren zur Abtrennung von Cyclohexanol aus aromatische Sulfonsäuren enthaltenden wäßrigen Lösungen durch Extraktion, wobei man als Extraktionsmittel unter Extraktionsbedingungen flüssige Cyclohexylether der Formel 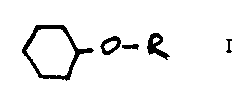
verwendet, in der R einen Alkylrest mit 1 bis 16 Kohlenstoffatomen, Cycloalkylrest mit 5 bis 8 Kohlenstoffatomen, Aralkylrest mit 7 bis 10 Kohlenstoffatomen oder Phenylrest, wobei die Reste zusätzlich unter Reaktionsbedingungen inerte Substituenten haben können, bedeutet.

Das neue Verfahren hat den Vorteil, daß die anzuwendende Menge an Extraktionsmittel erheblich vermindert wird und ein besseres Resultat bei der Extraktion erzielt wird. Dies gilt auch bei der Verwendung von keine Hydroxylgruppen enthaltenden Sulfonsäuren als Hydratisierungsmittel bei der Herstellung von Cyclohexanol, wodurch zusätzlich die Ausbeute an Cyclohexanol gesteigert wird.

Erfindungsgemäß geht man von wäßrigen Lösungen aus, die Cyclohexanol und aromatische Sulfonsäuren gelöst enthalten. Solche Lösungen erhält man bei der Hydratisierung von Cyclohexen zu Cyclohexanol, wobei man Cyclohexen in wäßrigen Lösungen, die 5 bis 80 Gew.% aromatische Sulfonsäuren insbesondere Benzol- oder Naphthalinsulfonsäuren, die substituiert sein können, wie Benzolsulfonsäure, Toluolsulfonsäure oder Naphthalinsulfonsäure, ferner Dodecylbenzolsulfonsäure enthalten bei einer Temperatur von 50 bis 200°C, insbesondere 70 bis 150°C, unter einem Druck von 1 bis 10 bar umsetzt. Vorteilhaft werden zusätzlich Molybdänsäure oder deren Salze, Vanadinoxid, Vanadate in einer Menge z.B. von 0,001 bis 5 Gew.%, bezogen auf die aromatische Sulfonsäure mitverwendet. Darüber hinaus ist es auch empfehlenswert, Heteropolysäuren wie Phosphormolybdänsäure, Phosphorwolframsäure, Phosphormolybdänwolframsäure oder Phosphormolybdänvanadinsäure mitzuverwenden. Geeignete Verfahren werden beispielsweise beschrieben in der europäischen Patentschrift 123 713 oder der europäischen Patentanmeldung 206 631. Aus dem so erhaltenen Reaktionsgemisch scheidet sich überschüssiges Cyclohexen, das geringere Mengen Cyclohexanol enthält, als organische Phase ab. Eine typische Zusammensetzung der erhaltenen wäßrigen Phase ist beispielsweise 5 bis 10 Gew.% Cyclohexanol, 40 bis 70 Gew.% aromatische Sulfonsäuren und 20 bis 50 Gew.% Wasser. Die Cyclohexenphase kann z.B. durch Dekantieren abgetrennt und getrennt aufgearbeitet werden. Für die Extraktion ist es jedoch nicht unbedingt erforderlich, Cyclohexen abzutrennen, da es als zusätzliches Extraktionsmittel zu verwenden ist.

Erfindungsgemäß werden die Cyclohexanol und aromatische Sulfonsäuren enthaltenden wäßrigen Lösungen mit mindestens einem Cyclohexyether der Formel I, in der R einen Alkylrest mit 1 bis 16 Kohlenstoffatomen, einen Cycloalkylrest mit 5 bis 8 Kohlenstoffatomen, einen Aralkylrest mit 7 bis 10 Kohlenstoffatomen oder einen Phenylrest, wobei die Reste unter den Reaktionsbedingungen inerte Substituenten haben können, bedeuten, extrahiert. Die als Extraktionsmittel verwendeten Cyclohexylether der Formel I sollen unter Extraktionsbedingungen flüssig sein. Es versteht sich, daß sie mit Wasser nicht oder nur wenig mischbar sind. Die unter R aufgeführten Reste können z.B. bis zu zwei inerte Substituenten wie Chloratome oder Alkoxygruppen enthalten. Bevorzugt hat der Rest R Kohlenwasserstoffstruktur.

Geeignete Ether sind beispielsweise Cyclohexylbenzylether, Cyclohexylalkylether oder Cyclohexylarylether. Besonders geeignet sind Cyclohexylether wie Cyclohexylmethylether, Cyclohexylethylether, Cyclohexyl-n-propylether, Cyclohexyl-i-propylether, Cyclohexyl-n-butylether, Cyclohexyl-i-butylether oder Phenylcyclohexylether.

In bevorzugten Cyclohexylethern der Formel I bezeichnet R einen Alkylrest mit 1 bis 12 Kohlenstoffatomen oder einen Cyclohexylrest. Besondere Bedeutung hat Dicyclohexylether erlangt.

Auf ein Volumenteil der zu extrahierenden Cyclohexanol und aromatische Sulfonsäuren enthaltenden wäßrigen Lösung wendet man zweckmäßig 0,1 bis 50 Volumenteile, insbesondere 1 bis 10 Volumenteile des Extraktionsmittels an. Die Extraktion wird im allgemeinen bei einer Temperatur von 0 bis 200°C, vorteilhaft von 10 bis 100°C, insbesondere 10 bis 50°C durchgeführt.

Die Extraktion der Cyclohexanol und aromatische Sulfonsäuren enthaltenden wäßrigen Lösung kann absatzweise durchgeführt werden, indem man die wäßrige Lösung intensiv mit dem Extraktionsmittel vermischt und anschließend die Phasen trennt. Vorteilhaft wird die Extraktion kontinuierlich im Gegenstrom in geeigneten Extraktionskolonnen wie Rührscheibenkolonnen, Pulsationskolonnen oder ein- oder mehrstufigen Mixer-Settler-Vorrichtungen durchgeführt. Die anfallende, aromatische Sulfonsäuren enthaltende wäßrige Phase wird wiederum für die Hydratisierung von Cyclohexen verwendet, während die Cyclohexanol enthaltende Extraktionsmittelphase durch Destillation aufgearbeitet wird. Falls in dem Cyclohexanol enthaltenden Extrakt geringe Mengen an aromatischen Sulfonsäuren enthalten sind, lassen sich diese durch Waschen mit wenig Wasser leicht entfernen und werden zweckmäßig der wäßrigen Lösung für die Hydratisierung zugefügt.

Besonders vorteilhaft haben die als Extraktionsmittel verwendeten Cyclohexylether I einen höheren Siedepunkt als Cyclohexanol. Dies führt dazu, daß lediglich das Cyclohexanol durch Destillation vom Extraktionsmittel abgetrennt werden muß. Das als Sumpfphase anfallende Extraktionsmittel wird zweckmäßig wiederum in die Extraktionsstufe zurückgeführt. In einer bevorzugten Ausführungsform wird die Extraktion unter Mitverwendung der bei der Hydratisierung anfallenden überschüssigen Cyclohexenphase durchgeführt. Bei der anschließenden Aufarbeitung erhält man somit zunächst eine Cyclohexenfraktion, die wiederum für die Hydratisierung verwendet wird, eine Cyclohexanolfraktion und als Sumpf das Extraktionsmittel.

Cyclohexanol, das nach dem Verfahren der Erfindung erhältlich ist, eignet sich zur Herstellung von Cyclohexanon, einem wichtigen Ausgangsstoff für Caprolactam.

Das Verfahren nach der Erfindung sei in folgenden Beispielen veranschaulicht. Die Gewichtsteile verhalten sich zu den Volumenteilen wie kg zu Liter.

### Beispiel 1

In einem Gefäß wurden 17,4 Volumenteile einer wäßrigen Lösung aus 54 Gew.%-Teilen p-Toluolsulfonsäure, 10 Gew.%-Teilen Cyclohexanol und 36 Gew.%-Teilen Wasser mit 21,5 Vol.-Teilen Dicyclohexylether für 5 Minuten bei Raumtemperatur intensiv gemischt. Nach Trennung der Phasen wurde gaschromatographisch ermittelt, daß 19,4 Gew.% des eingesetzten Cyclohexanols aus der wäßrigen Phase extrahiert worden waren.

### Vergleichsbeispiele 1 - 5

Die Extraktion wurde wie im Beispiel 1 angegeben durchgeführt, jedoch wurden zur Extraktion jeweils die in der Tabelle angegebenen Kohlenwasserstoffe verwendet. Die Ergebnisse sind in der Tabelle wiedergegeben:

**Tabelle**

| Vergleichsbeispiel | Extraktionsmittel | Extrahiertes Cyclohexanol [Gew.-%] |
|---|---|---|
| 1 | Cyclohexen | 13,7 |
| 2 | Toluol | 16,4 |
| 3 | Tetralin | 14,4 |
| 4 | Decalin | 5,2 |
| 5 | 4-tert.-Butyltoluol | 11,6 |

## Patentansprüche

1. Verfahren zur Abtrennung von Cyclohexanol aus solches und aromatische Sulfonsäuren enthaltenden wäßrigen Lösungen durch Extraktion, dadurch gekennzeichnet, daß man als Extraktionsmittel unter Extraktionsbedingungen flüssige Cyclohexylether der Formel 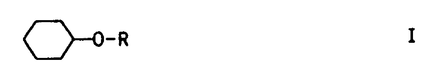 verwendet, in der R einen Alkylrest mit 1 bis 16 Kohlenstoffatomen, Cycloalkylrest mit 5 bis 8 Kohlenstoffatomen, Aralkyrest mit 7 bis 10 Kohlenstoffatomen oder Phenylrest, wobei die Reste zusätzlich unter den Reaktionsbedingungen inerte Substituenten haben können, bedeutet.

2. Verfahren nach Anspruch 1, dadurch gekennzeichnet, daß man Cyclohexylether der Formel I, in der R einen Alkylrest mit 1 bis 12 Kohlenstoffatomen oder einen Cyclohexylrest bedeutet, verwendet.

3. Verfahren nach den Ansprüchen 1 und 2, dadurch gekennzeichnet, daß man Dicyclohexylether verwendet.

4. Verfahren nach den Ansprüchen 1 bis 3, dadurch gekennzeichnet, daß man je Volumenteil wäßrige Lösung 1 bis 10 Volumenteile Extraktionsmittel der Formel I verwendet.

5. Verfahren nach den Ansprüchen 1 bis 4, dadurch gekennzeichnet, daß man die Extraktion bei einer Temperatur von 10 bis 100°C durchführt.

6. Verfahren nach den Ansprüchen 1 bis 5, dadurch gekennzeichnet, daß man eine wäßrige Lösung, die durch Hydratisierung von Cyclohexen in einer wäßrigen Lösung einer aromatischen Sulfonsäure erhalten wurde, ohne Abtrennung des überschüssigen Cyclohexens für die Extraktion verwendet.

## Claims

1. A process for removing cyclohexanol from an aqueous solution containing it and an aromatic sulfonic acid by extraction, which comprises extracting cyclohexyl ether liquid under extraction conditions and having the formula 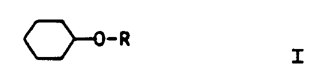 where R is alkyl of from 1 to 16 carbon atoms, cycloalkyl of from 5 to 8 carbon atoms, aralkyl of from 7 to 10 carbon atoms or phenyl, which may each additionally have substituents which are inert under the reaction conditions.

2. A process as claimed in claim 1, wherein a cyclohexyl ether of the formula I where R is alkyl of from 1 to 12 carbon atoms or cyclohexyl is used.

3. A process as claimed in claims 1 and 2, wherein dicyclohexyl ether is used.

4. A process as claimed in claims 1 to 3, wherein from 1 to 10 parts by volume of extractant of the formula I are used per part by volume of aqueous solution.

5. A process as claimed in claims 1 to 4, wherein the extraction is carried out at from 10 to 100°C.

6. A process as claimed in claims 1 to 5, wherein an aqueous solution obtained by hydration of cyclohexene in an aqueous solution of an aromatic sulfonic acid is used for the extraction without removal of the excess cyclohexene.

## Revendications

1. Procédé de séparation de cyclohexanol à partir de solutions aqueuses contenant celui-ci et des acides sulfoniques aromatiques par extraction, caractérisé en ce que l'on utilise comme agent d'extraction dans les conditions d'extraction un oxyde de cyclohexyle liquide de formule 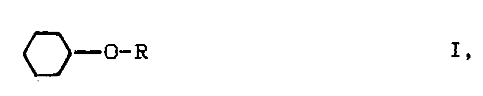 dans laquelle R est un reste alkyle ayant 1 à 16 atomes de carbone, un reste cycloalkyle ayant 5 à 8 atomes de carbone, un reste aralkyle ayant 7 à 10 atomes de carbone ou un reste phényle, les restes pouvant comporter de plus dans les conditions de la réaction des substituants inertes.

2. Procédé selon la revendication 1, caractérisé en ce que l'on utilise un oxyde de cyclohexyle de formule I, dans laquelle R est un reste alkyle ayant 1 à 12 atomes de carbone ou un reste cyclohexyle.

3. Procédé selon la revendication 1 ou 2, caractérisé en ce que l'on utilise l'oxyde de dicyclohexyle.

4. Procédé selon l'une des revendications 1 à 3, caractérisé en ce que l'on utilise, par partie en volume de la solution aqueuse, 1 à 10 parties en volume de l'agent d'extraction de formule I.

5. Procédé selon l'une quelconque des revendications 1 à 4, caractérisé en ce que l'on effectue l'extraction à une température comprise entre 10 et 100°C.

6. Procédé selon l'une quelconque des revendications 1 à 5, caractérisé en ce que l'on utilise, pour l'extraction, une solution aqueuse, qui a été obtenue par hydratation de cyclohexène dans une solution aqueuse d'un acide sulfonique aromatique, sans séparation du cyclohexène en excès.
